# EUROPÄISCHE PATENTSCHRIFT

(11) **EP 3 285 823 B1**
(45) Veröffentlichungstag und Bekanntmachung des Hinweises auf die Patenterteilung: **17.02.2021**
(21) Anmeldenummer: 16722775.0
(22) Anmeldetag: 18.03.2016
(51) Int. Cl.: A61M 1/10

(54) **VERFAHREN ZUM FÖRDERN EINES MEDIUMS MIT EINER PUMPE UND PUMPE MIT EINEM ROTOR, EINEM GEHÄUSE UND EINEM ANTRIEB**
METHOD FOR CONVEYING A MEDIUM WITH A PUMP AND PUMP COMPRISING A ROTOR, A HOUSING AND A DRIVE
PROCÉDÉ DE REFOULEMENT D'UN FLUIDE AVEC UNE POMPE ET POMPE POURVUE D'UN ROTOR, D'UN CARTER ET D'UN MÉCANISME D'ENTRAÎNEMENT

(30) Priorität: 20.04.2015 DE 102015004968
(43) Veröffentlichungstag der Anmeldung: 28.02.2018
(73) Patentinhaber: Xenios AG, 74076 Heilbronn (DE)
(72) Erfinder: FILIPON, Sven, 74080 Heilbronn (DE); BRÜCKNER, Benjamin, 74078 Heilbronn (DE); WAGNER, Ozan, 74254 Offenau (DE)
(74) Vertreter: Graf von Stosch, Andreas
(86) Internationale Anmeldenummer: PCT/DE2016/000118
(87) Internationale Veröffentlichungsnummer: WO 2016/169540

(56) Entgegenhaltungen:
- EP-A1- 0 665 024
- WO-A2-98/14225
- WO-A2-2004/082467

## Beschreibung

Die Erfindung betrifft ein Verfahren zum Fördern von Blut oder Primingflüssigkeit als Medium mit einer Blutpumpe und eine Pumpe mit einem Rotor, einem Gehäuse und einem Antrieb.

Pumpen, wie insbesondere Zentrifugalpumpen oder Diagonalpumpen werden in der Medizintechnik zur Förderung von Blut eingesetzt. Diese haben gegenüber Rollerpumpen den wesentlichen Vorteil, dass sie blutschonend sind. Ein Nachteil bei Zentrifugalpumpen liegt darin, dass sie Luft, die in die Zentrifugalpumpe gelangt, nicht weiterbefördern kann. Die Luft verfängt sich meistens im Rotor und beeinträchtigt die Leistung der Zentrifugalpumpe. Es können sogar Überhitzungen am Rotorlager entstehen, die zu einer Schädigung der Blutpumpe und des Blutes führen können.

Für große Kreiselpumpen wird in Johann Friederich Güllich: Kreiselpumpen. 3. Auflage. Heidelberg: Springer, 2010. 298, 680 - 681, 772 - 780. - ISBN 978-3-642-05478-5 beschrieben, dass die Pumpe auf einen bestimmten Förderstrom geregelt werden kann und dann bei Gasblasen zwangsläufig die Förderleistung erhöht. Eine derartige Regelung würde bei einer Blutpumpe dazu führen, dass bei Gas in der Pumpe sich die Förderleistung erhöht und das Blut schädigt.

Der Erfindung liegt die Aufgabe zugrunde, ein Verfahren zur Vermeidung einer Ansammlung von Gas beim Fördern eines Mediums mit einer Blutpumpe wie insbesondere einer Zentrifugalpumpe oder eine Diagonalpumpe vorzuschlagen, das dazu führt, dass derartige Schädigungen ausgeschlossen werden. Außerdem liegt der Erfindung die Aufgabe zugrunde, eine gattungsgemäße Pumpe so weiterzuentwickeln, dass derartige Beeinträchtigungen nicht mehr zu befürchten sind.

Diese Aufgabe wird in verfahrenstechnischer Hinsicht mit den Merkmalen des Patentanspruchs 1 und in vorrichtungsmäßiger Hinsicht mit dem Merkmal des Patentanspruchs 18 gelöst.

Es hat sich herausgestellt, dass bereits eine kurzzeitige Erhöhung der Leistung der Pumpe durch eine Erhöhung der Drehzahl dazu ausreicht, die Luftblasen, die sich im Bereich des Rotorlagers sammeln, nach außen zu schleudern, sodass sie mit der Strömung des Blutes aus der Pumpe herausgefördert werden. Daher wird vorgeschlagen, dass im geförderten Medium das Gas gemessen wird und die Leistung der Pumpe abhängig vom gemessenen Gas nur kurzzeitig erhöht wird. Dies ermöglicht es, den Gaseintrag, der zu einem Leistungsabfall oder zu Pumpenschädigungen führen könnte, frühzeitig zu erkennen und mittels einer kurzzeitigen Geschwindigkeitserhöhung über die Beschleunigung der Pumpenflügel sich ansammelnde Gasblasen nach außen zu schleudern oder bereits eine Ansammlung von Gas zu vermeiden.

Das erfindungsgemäße Verfahren kann für jede Pumpe eingesetzt werden, bei der Flüssigkeit durch einen Rotor angetrieben von einem Zulauf zu einem Ablauf gefördert wird. Bevorzugt dient das Verfahren für den Einsatz an einer Zentrifugalpumpe oder einer Diagonalpumpe.

Besonders vorteilhaft ist ein pulsatiler Modus an der Pumpe bzw. im Blutfluss. Dies führt dazu, dass die Luft im kurzen Augenblick des abgesenkten Volumenstroms die Möglichkeit hat, sich vom Rotor zu lösen. Der anschließende Stoß durch einen erhöhten Blutstrom fördert die Luftblase dann durch den Blutauslass aus der Pumpe heraus. Dadurch wird die Pumpe auf einfache Art und Weise luftfrei.

Um frühzeitig auf einen Gaseintrag reagieren zu können, kann das Gas in einer Zuleitung zur Pumpe gemessen werden. Die Messeinrichtung kann auf einen speziellen Durchmesser von Gasblasen eingestellt werden, der die Gefahr birgt, dass sich Luftblasen mit diesem Durchmesser in der Blutpumpe festsetzen. Außerdem kann die Messung kumulativ über einen Zeitraum die zur Blutpumpe geführten Blasenvolumina addieren, um ab einem vorbestimmten Grenzwert die Leistung der Pumpe kurzzeitig zu erhöhen, sodass die in der Pumpe angesammelte Luft aus der Pumpe herausbefördert wird.

Alternativ oder kumulativ wird vorgeschlagen, dass das Gas in oder an der Pumpe, beispielsweise am Pumpengehäuse, gemessen wird. Dies erleichtert es, die tatsächlich in der Pumpe festsitzende Gasmenge zu ermitteln, um bei Überschreiten einer kritischen Menge darauf durch Erhöhung der Pumpenleistung zu reagieren.

Die Erhöhung der Pumpenleistung führt zu besonderen Zentrifugalkräften, die nicht nur das Blut sondern auch in der Blutpumpe angesammeltes Gas radial nach außen schleudert und mit dem strömenden Blut mitreißt. Hierzu reicht es in der Regel aus, dass die Pumpenleistung nur kurzzeitig erhöht wird. Als Blutpumpe wird daher eine Pumpe vorgeschlagen, die etwa 0 bis 8 l/min bei einer Drehzahl von maximal 10.000 l/min fördert.

Versuche haben gezeigt, dass es ausreicht, wenn die Pumpenleistung für eine Zeitspanne von weniger als 5 s und vorzugsweise von bis zu 2 s erhöht wird. Jegliche Leistungserhöhung führt dazu, dass die Zeitspanne der Leistungserhöhung größer als 0 s ist. In der Praxis liegt die Zeitspanne der Leistungserhöhung bei über 0,1 s und etwa bei 0,1 bis 2 s.

Neben der Dauer der erhöhten Leistung ist vor allem auch die Steigung des Anstiegs der Pumpenleistung relevant, um durch die Erhöhung des Volumenstroms innerhalb möglichst kurzer Zeit die Gasansammlung aus der Zentrifugalpumpe zu entfernen. Daher wird vorgeschlagen, dass die Pumpenleistung innerhalb einer Zeit von weniger als 5 s und vorzugsweise von bis zu 2 s um bis zu 100 % erhöht wird. Das heißt, die Pumpenleistung sollte vorzugsweise innerhalb einer Zeit von weniger als 2 s auf die doppelte Leistung erhöht werden, um durch die starke Beschleunigung das Blut aus der Pumpe zu fördern.

Insbesondere um derartige Beschleunigungen zu erzielen, ohne das geförderte Medium durch einen zu hohen Leistungseintrag zu schädigen, wird vorgeschlagen, dass die Pumpenleistung vor der Erhöhung abgesenkt wird. Eine kurzzeitige Absenkung vor der Erhöhung ermöglicht eine schonende Leistungssteigerung für einen kurzen Zeitraum. Jegliche Leistungsabsenkung führt dazu, dass die Zeitspanne der Leistungsabsenkung größer als 0 s ist. In der Praxis liegt die Zeitspanne der Leistungsabsenkung bei über 0,1 s und etwa bei 0,1 bis 2 s.

Entsprechend der Dynamik bei der Erhöhung der Leistung ist es auch vorteilhaft, wenn die Leistung vor der Erhöhung schnell abgesenkt wird. Daher wird vorgeschlagen, dass die Pumpenleistung vor der Erhöhung innerhalb einer Zeit von weniger als 5 s und vorzugsweise von bis zu 2 s um bis zu 100 % abgesenkt wird. Dadurch entsteht ein kurzzeitiger oder zeitlich begrenzter pulsatiler Betrieb

Eine einfache verfahrenstechnische Regelung sieht vor, dass die Pumpenleistung bei einer Detektion eines Gaseintrags erhöht wird. Dies kann jedoch dazu führen, dass die Pumpenleistung bei vielen kleinen Gasblasen in schnellem Abstand hintereinander immer wieder erhöht wird. Daher wird als vorteilhafte Ausführungsvariante vorgeschlagen, dass die Pumpenleistung nach einer Detektion einer vorgegebenen Gasmenge erhöht wird. Diese vorgegebene Gasmenge kann eine einmalig durch nur eine Gasblase zugeführte Gasmenge sein oder eine im Rahmen eines längeren Messverfahrens aufgelaufene aufaddierte Gasmenge.

Insbesondere wenn die Detektion beabstandet zur Blutpumpe in einer Zuleitung vorgenommen wird, ist es vorteilhaft, wenn die Pumpenleistung nach einer Zeitspanne erhöht wird, die der Zeit entspricht, die das Medium benötigt, um vom Detektor zum Rotor der Pumpe zu gelangen. Die Beschleunigung des Rotors wird somit etwa in dem Moment vorgesehen, in dem die Gasblase in den Bereich des Rotors gelangt.

Um ein Ansammeln von Gas in der Pumpe zu ermöglichen und um die Häufigkeit des pulsatilen Einsatzes der Blutpumpe zu reduzieren, wird vorgeschlagen, dass die Pumpenleistung erst nach Ablauf einer vorgegebenen auf den ersten detektieren Gaseintrag folgenden Zeitspanne erhöht wird. Diese Zeitspanne kann beispielsweise größer als 3 s sein und bei etwa 5 bis 30 s liegen.

Zum Austreiben einer Gasblase aus der Pumpe kann eine einmalige Erhöhung oder vorzugsweise eine einmalige Absenkung mit darauffolgender Erhöhung des Volumenstroms ausreichen. Vorteilhaft ist es jedoch, wenn dieser Vorgang mehrmals hintereinander vorgenommen wird. In diesem Fall wird von gepulster Pumpenleistung gesprochen. Daher ist es vorteilhaft, wenn die Pumpenleistung über eine Zeit von mehr als 3 s, vorzugsweise mehr als 5 s gepulst ist.

Wenn bei einer Pumpe die Pumpenleistung gepulst variiert wird, spricht man von einem pulsatilen Betrieb. Gerade während der Füllung der Pumpe mit einem flüssigen Medium besteht eine erhöhte Gefahr des Gaseintrags. Daher wird vorgeschlagen, dass die Pumpe während des Priming pulsatil betrieben wird.

Es gibt verschiedene Verfahren zur Detektion des Gases innerhalb der Pumpe oder einer Zuleitung. Um einen Gas oder Lufteinschluss zu detektieren eignen sich beispielsweise kapazitive Sensoren, Widerstandssensoren, Thermoelemente, Multimeter, Ultraschallsensoren oder magnetinduktive Sensoren. Besonders geeignet sind Sensoren, die berührungslos messen. Der Detektor sollte mindestens 0,2 ml und vorzugsweise mindestens 0,1 ml wie beispielsweise 0,5 ml detektieren, um für den Einsatz in Verbindung mit der Blutpumpe besonders gut geeignet zu sein.

Ein indirektes Verfahren zur Detektion des Gases wird bereitgestellt, indem die Leistungsaufnahme der Pumpe in Relation zu deren Leistungsabgabe gemessen wird. Wenn beispielsweise der Volumenstrom des geförderten Mediums abnimmt und die Stromaufnahme an der Pumpe ansteigt, ist dies ein Zeichen dafür, dass sich Gas innerhalb der Pumpe angesammelt hat. Da in der Regel der Volumenstrom konstant gehalten werden soll, reicht es aus, wenn eine Erhöhung der Leistungsaufnahme der Pumpe detektiert wird, um auf eine Gasansammlung innerhalb der Pumpe zurückzuschließen.

Alternativ oder kumulativ wird vorgeschlagen, dass das Gas dadurch detektiert wird, dass die Temperatur am Rotorlager gemessen wird. Auch eine Erhöhung der Temperatur am Rotorlager weist auf eine Gasansammlung hin.

Konstruktiv wird die der Erfindung zugrunde liegende Aufgabe mit einer Zentrifugalpumpe mit einem Rotor, einem Gehäuse und einem Antrieb gelöst, die einen Gasdetektor aufweist, der bei Detektion von Gas auf den Antrieb wirkt.

Dieser Detektor kann in einer Zuleitung der Pumpe angeordnet sein oder im oder am Gehäuse der Pumpe. Alternativ oder kumulativ wird vorgesehen, dass der Detektor am Rotor oder an der Rotorlagerung angeordnet ist.

Eine vorteilhafte Ausgestaltung des Detektors sieht vor, dass der Detektor ein kapazitiver Sensor ist. Derartige Sensoren können Gas im flüssigen Medium messen, ohne mit dem Medium in Berührung zu geraten.

Vorteilhaft ist es, wenn Sensor und Blutpumpe derart geschaltet sind, dass bei defektem Sensor die Pumpe im Normalbetrieb angetrieben ist, sodass die Pumpe bei einem defekten Sensor nicht dauerhaft pulsatil betrieben wird. Dies wird dadurch erreicht, dass der Sensor beim Fördern des Mediums kein Signal sendet und bei Lufteintrag ein Signal sendet.

Verschiedene Ausführungsbeispiele erfindungsgemäßer Blutpumpen sind in der Zeichnung dargestellt und werden im Folgenden näher beschrieben. Es zeigt
- Figur 1: schematisch einen Pumpenkopf mit Regelung,
- Figur 2: schematisch eine Draufsicht auf den in Figur 1 gezeigten Pumpenkopf und
- Figur 3: schematisch einen Schnitt durch den in Figur 1 gezeigten Pumpenkopf.

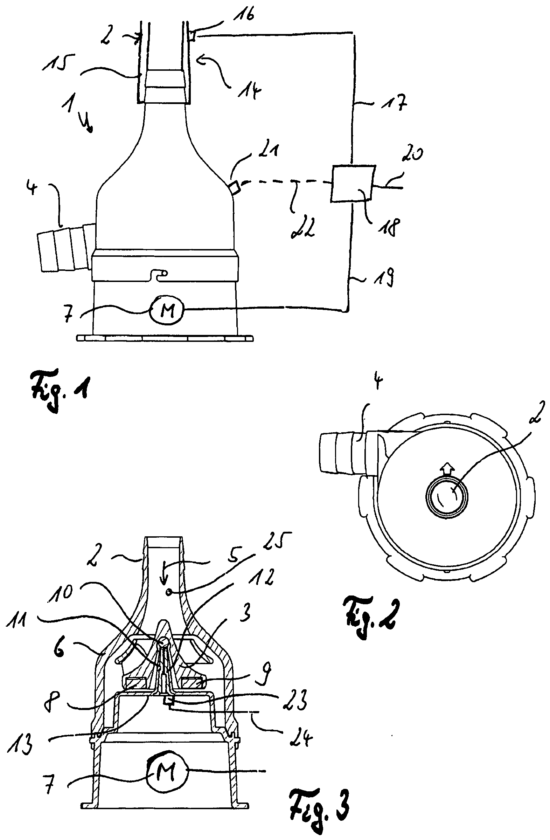

Die in Figur 1 gezeigte Pumpe 1 ist eine Diagonalpumpe, da das Fördermedium schräg zur Pumpenwelle aus dem Laufrad austritt. Je nach Konstruktion der Pumpe kann der Förderwinkel des Laufrades variiert werden, sodass die Pumpe im gesamten Spektrum von radial bis axial ausgeführt werden kann, um auf die jeweilige Anwendung zugeschnitten zu werden. Die Pumpe 1 hat jedoch einen überwiegenden radialen Förderanteil, um zentral am Einlass 2 eintretendes Blut mittels des Rotors 3 zum Auslass 4 zu fördern.

Dabei fließt das Blut 5 durch das Gehäuse 6, während ein Antrieb 7 berührungslos auf Magnete 8, 9 des Rotors 3 wirkt, um den Rotor 3 zu bewegen. Der Rotor 3 ist mit einer Kugel 10 auf einem Stift 11 gelagert. Im Stift 11 ist ein Metallkegel 12 angeordnet, der die Wärme von der Kugel 10 zur Basisfläche 13 des Gehäuses ableitet.

Der Bluteinlass 2 bildet die Zuleitung 14, die im vorliegenden Fall als Schlauch 15 ausgebildet ist. An diesem Schlauch 15 ist ein kapazitiver Schaltsensor 16 als Detektor vorgesehen. Dieser kapazitive Schaltsensor 16 steht über eine Leitung 17 mit einer Pumpensteuerung 18 in Verbindung, die wiederum über eine Leitung 19 mit dem Antrieb 7 des Rotors 3 in Verbindung steht. Außerdem hat die Steuerung 18 eine weitere Leitung 20, um die Werte der Steuerung an eine übergeordnete Einrichtung (nicht gezeigt) zu melden und von dort Regelungs- und Steuerungsparameter entgegenzunehmen. Die Steuerung ist beispielsweise in einer Pumpenkonsole vorgesehen.

Ein optionaler weiterer kapazitiver Schaltsensor 21 misst als Detektor am Pumpengehäuse 6 einen Lufteintrag, um ihn über die Leitung 22 an die Steuerung 18 zu melden.

Als dritter optionaler Detektor dient eine Temperaturmesseinrichtung 23 an der Basisfläche 13 des Gehäuses 6, der die Temperatur am Lager 10, 11 misst, indem er am metallelen Kegel 12 die Temperatur abgreift. Auch dieser Wert kann über eine Leitung 24 an die Steuerung 18 weitergegeben werden.

Wenn mit dem Medium, das im vorliegenden Fall Blut 5 ist, eine Gasblase 25 in das Gehäuse 6 der Blutpumpe 1 gelangt, kann diese Gasblase 25 bereits beim Durchlauf durch den Schlauch 15 vom Detektor 16 ermittelt worden seien. Eine weitere Möglichkeit, diese Gasblase 15 zu entdecken, bietet der weitere kapazitive Schaltsensor 21. Wenn sich Gasblasen zentral im Bereich der Achse der Lagerung des Rotors 3 ansammelt, sinkt dadurch die Pumpenleistung, die die Steuerung 18 ermitteln kann. Außerdem verändert sich die Temperatur an der Lagerung, die mit der Temperaturmesseinrichtung 23 ermittelt wird.

Im vorliegenden Fall kann es zu einer Luftansammlung zwischen dem Stift 11 und dem inneren Bereich des Rotors 3 kommen, die dort mittels eines kapazitiven Sensors ermittelt werden kann. Eine dort eingefangene Luftblase kann sich derart verfangen, dass sie nicht mehr zum Auslass 4 weiter gefördert wird.

Wenn nun ein pulsatiler Modus bzw. Blutfluss mittels der Regelung 18 am Antrieb 7 eingeschaltet wird, hat die Luft im kurzen Augenblick des niedrigen Flusses die Möglichkeit sich vom Rotor zu lösen. Der anschließende Stoß fördert die Luftblase dann durch den Blutauslass 4 aus der Pumpe 1 heraus. Dadurch wird die Blutpumpe wieder luftfrei. Vorteilhaft ist eine Absenkung über 0,1 bis 2 s und dann eine Erhöhung über 0,1 bis 2 s für eine Gesamtdauer des pulsatilen Betriebs von ca. 5 bis 30 s.

In der Praxis besteht meistens in der vom Auslass 4 fortführenden Leitung (nicht gezeigt) die zum Patienten führt, bereits ein Flusssensor mit integriertem Blasendetektor, beispielswiese als Clamp-On Transducer IPX4. Um den Patienten nicht zu gefährden, sollte dieser Sensor weiterhin zum Patienten hin positioniert sein. Ein zweiter Sensor kann dann direkt vor der Pumpe 1 positioniert sein. Wenn dieser eine Luftblase erkennt, kann das System eine fest eingestellte Pulsatilität erzeugen, um die Luft am Rotor aus dem Gehäuse zu fördern. In der Regel wird das Blut mit der Luft dann weiter zu einem Oxygenator geleitet, wo die Luft letztendlich entweichen kann. Die Pulsatilität zum Austreiben der Luft wird in dem Ausführungsbeispiel auf 10 Sekunden eingestellt.

Alternativ zu einem am Zuleitungsschlauch vorgesehenen Clamp-On-Sensor kann ein kapazitiver Sensor 16 auf der Oberfläche des Pumpengehäuses positioniert werden. Derartige kapazitive Schalter detektieren die Änderung der Dielektrizitätskonstante und führen diese Änderung in ein Schaltsignal über. Wenn beispielsweise mehr als 5 Sekunden Luft in dem Bereich des Rotors vorliegt, kann eine pulsatile Betriebsweise der Pumpe 1 durchgeführt werden. Dabei sollte die Schaltfunktion so sein, dass der Sensor bei Luft aktiv ist und bei normalem Betrieb aus (Schaltfunktion "Öffner" YES). Dadurch wird dem Risiko entgegengewirkt, dass der Sensor bei einem Defekt dauerhaft pulsiert.

## Patentansprüche

1. Verfahren zur Vermeidung einer Ansammlung von Gas beim Fördern von Blut oder Primingflüssigkeit als Medium mit einer Blutpumpe (1), bei dem in einer Zuleitung zur Pumpe, in oder an der Pumpe im Medium (5) Gas (25) gemessen wird und die Pumpenleistung abhängig vom gemessenen Gas nur kurzzeitig erhöht wird.

2. Verfahren nach Anspruch 1, ***dadurch gekennzeichnet, dass*** die Pumpe (1) eine Zentrifugalpumpe oder eine Diagonalpumpe ist.

3. Verfahren nach einem der vorhergehenden Ansprüche, ***dadurch gekennzeichnet, dass*** die Pumpenleistung für eine Zeitspanne von weniger als 5 s und vorzugsweise von 0 bis 2 s und insbesondere von 0,1 bis 2 s erhöht wird.

4. Verfahren nach einem der vorhergehenden Ansprüche, ***dadurch gekennzeichnet, dass*** die Pumpenleistung innerhalb einer Zeit von weniger als 5 s und vorzugsweise von 0 bis 2 s und insbesondere von 0,1 bis 2 s um bis zu 100 % erhöht wird.

5. Verfahren nach einem der vorhergehenden Ansprüche, ***dadurch gekennzeichnet, dass*** die Pumpenleistung vor der Erhöhung abgesenkt wird.

6. Verfahren nach einem der vorhergehenden Ansprüche, ***dadurch gekennzeichnet, dass*** die Pumpenleistung vor der Erhöhung innerhalb einer Zeit von weniger als 5 s und vorzugsweise von 0 bis 2 s und insbesondere von 0,1 bis 2 s um bis zu 100 % abgesenkt wird.

7. Verfahren nach einem der vorhergehenden Ansprüche, ***dadurch gekennzeichnet, dass*** die Pumpenleistung bei einer Detektion eines Gaseintrags erhöht wird.

8. Verfahren nach einem der vorhergehenden Ansprüche, ***dadurch gekennzeichnet, dass*** die Pumpenleistung nach einer Detektion einer vorgegebenen Gasmenge erhöht wird.

9. Verfahren nach einem der vorhergehenden Ansprüche, ***dadurch gekennzeichnet, dass*** die Pumpenleistung nach einer Zeitspanne erhöht wird, die der Zeit entspricht, die das Medium (5) benötigt, um vom Detektor (16) zum Rotor (3) der Pumpe (1) zu gelangen.

10. Verfahren nach einem der vorhergehenden Ansprüche, ***dadurch gekennzeichnet, dass*** die Pumpenleistung nach Ablauf einer vorgegebenen auf den ersten detektierten Gaseintrag folgenden Zeitspanne erhöht wird. (z.B. Zeitspanne größer 3 s)

11. Verfahren nach einem der vorhergehenden Ansprüche, ***dadurch gekennzeichnet, dass*** die Pumpenleistung über eine Zeit von mehr als 3, vorzugsweise mehr als 5 Sekunden gepulst ist.

12. Verfahren nach einem der vorhergehenden Ansprüche, ***dadurch gekennzeichnet, dass*** die Pumpe (1) während des Priming pulsatil betrieben wird.

13. Verfahren nach einem der vorhergehenden Ansprüche, ***dadurch gekennzeichnet, dass*** das Gas (25) detektiert wird, indem die Leistungsaufnahme der Pumpe (1) in Relation zu deren Leistungsabgabe gemessen wird.

14. Verfahren nach einem der vorhergehenden Ansprüche, ***dadurch gekennzeichnet, dass*** das Gas (25) detektiert wird, indem die Temperatur am Lager (10, 11) des Rotors (3) gemessen wird.

15. Blutpumpe (1), insbesondere Zentrifugalpumpe oder Diagonalpumpe, mit einem Rotor (3), einem Gehäuse (6) und einem Antrieb (7), ***dadurch gekennzeichnet, dass*** sie einen Gasdetektor (16, 21, 23) aufweist, der bei Detektion von Gas (25) auf den Antrieb (7) wirkt, um die Pumpenleistung abhängig vom gemessenen Gas nur kurzzeitig zu erhöhen.

16. Blutumpe nach Anspruch 15, ***dadurch gekennzeichnet, dass*** der Detektor (16) in einer Zuleitung (2) zur Pumpe (1) angeordnet ist.

17. Blutpumpe nach einem der vorhergehenden Vorrichtungsansprüche, ***dadurch gekennzeichnet, dass*** der Detektor (21) im oder am Gehäuse (6) der Pumpe (1) angeordnet ist.

18. Blutpumpe nach einem der vorhergehenden Vorrichtungsansprüche, ***dadurch gekennzeichnet, dass*** der Detektor (23) am Rotor (3) oder an der Rotorlagerung (10, 11) angeordnet ist.

19. Blutpumpe nach einem der vorhergehenden Vorrichtungsansprüche, ***dadurch gekennzeichnet, dass*** der Detektor (16, 21) ein berührungslos messender Sensor ist.

20. Blutpumpe nach einem der vorhergehenden Vorrichtungsansprüche, ***dadurch gekennzeichnet, dass*** der Sensor des Detektors (16, 21, 23) beim Fördern des Mediums (5) kein Signal sendet und bei Lufteintrag ein Signal sendet.

## Claims

1. Method for preventing accumulation of gas when conveying blood or priming fluid as medium with a blood pump (1), in which gas (25) is measured in the medium (5) in an inlet line to the pump, in or on the pump and the pump output is increased only briefly as a function of the measured gas.

2. Method according to claim 1 **characterised in that** the pump (1) is a centrifugal pump or a diagonal pump.

3. Method according to any one of the preceding claims **characterised in that** the pump output is increased for a period of less than 5 seconds and preferably from 0 to 2 seconds, more particularly from 0.1 to 2 seconds.

4. Method according to any one of the preceding claims **characterised in that** the pump output is increased by up to 100% within a period of less than 5 seconds and preferably 0 to 2 seconds, more particularly from 0.1 to 2 seconds.

5. Method according to any one of the preceding claims **characterised in that** the pump output is reduced before the increase.

6. Method according to any one of the preceding claims **characterised in that** before the increase the pump output is reduced by up to 100% within a period of less than 5 seconds and preferably 0 to 2 seconds, more particularly from 0.1 to 2 seconds.

7. Method according to any one of the preceding claims **characterised in that** the pump output is increased on detection of gas entry.

8. Method according to any one of the preceding claims **characterised in that** the pump output is increased after the detection of a predetermined gas quantity.

9. Method according to any one of the preceding claims **characterised in that** the pump output is increased after a period of time corresponding to the time required by the medium (5) to travel from the detector (16) to the rotor (3) of the pump (1).

10. Method according to any one of the preceding claims **characterised in that** the pump output is increased on expiry of a predetermined period of time following the initially detected gas entry (e.g. period or time greater than 3 seconds).

11. Method according to any one of the preceding claims **characterised in that** the pump output is pulsed over a period or more than 3, preferably more than 5 seconds.

12. Method according to any one of the preceding claims **characterised in that** the pump (1) is operated in a pulsatile manner during priming.

13. Method according to any one of the preceding claims **characterised in that** the gas (25) is detected **in that** the power uptake of the pump (1) is measured in relation to its power output.

14. Method according to any one of the preceding claims **characterised in that** the gas (25) is detected **in that** the temperature on the bearing (10, 11) of the rotor (3) is measured.

15. Blood pump (1), in particular centrifugal pump or diagonal pump, with a rotor (3), a housing (6) and an actuator (7) **characterised in that** it comprises a gas detector (16, 21, 23) which acts on the actuator (7) when gas (25) is detected.

16. Blood pump according to claim 15 **characterised in** the detector (16) is arranged in an inlet line (2) to the pump (1).

17. Blood pump according to any one of the preceding device claims **characterised in that** the detector (21) is arranged in or on the housing (6) of the pump (1).

18. Blood pump according to any one of the preceding device claims **characterised in that** the detector (23) is arranged on the rotor (3) or on the rotor bearing (10, 11).

19. Blood pump according to any one of the preceding device claims **characterised in that** the detector (16, 21) is a contactlessly measuring sensor.

20. Blood pump according to any one of the preceding device claims **characterised in that** the sensor of the detector (16, 21, 23) does not send a signal during the conveying of the medium (5) and sends a signal in the event of air entry.

## Revendications

1. Procédé de prévention d'une accumulation de gaz lors du transfert de sang ou de liquide de priming comme support, comportant une pompe à sang (1), dans lequel dans une alimentation vers la pompe, on mesure du gaz (25) dans le milieu (5) dans ou sur la pompe, la puissance de la pompe n'étant augmentée que temporairement en fonction du gaz mesuré.

2. Procédé selon la revendication 1, **caractérisé en ce que** la pompe (1) est une pompe centrifuge ou une pompe diagonale.

3. Procédé selon l'une des revendications précédentes, **caractérisé en ce que** l'augmentation de la puissance de la pompe se fait pendant une période inférieure à 5 s, de préférence comprise entre 0 et 2 s et notamment comprise entre 0,1 et 2 s.

4. Procédé selon l'une des revendications précédentes, **caractérisé en ce que** l'augmentation de la puissance de la pompe se fait de jusqu'à 100 % pendant une période inférieure à 5 s, de préférence comprise entre 0 et 2 s et notamment comprise entre 0,1 et 2 s.

5. Procédé selon l'une des revendications précédentes, **caractérisé en ce que** la puissance de la pompe est abaissée avant l'augmentation.

6. Procédé selon l'une des revendications précédentes, **caractérisé en ce que** l'abaissement de la puissance de la pompe se fait de jusqu'à 100 % avant l'augmentation pendant une période inférieure à 5 s, de préférence comprise entre 0 et 2 s et notamment comprise entre 0,1 et 2 s.

7. Procédé selon l'une des revendications précédentes, **caractérisé en ce que** la puissance de la pompe est augmentée lors de la détection d'une entrée de gaz.

8. Procédé selon l'une des revendications précédentes, **caractérisé en ce que** la puissance de la pompe est augmentée par suite de la détection d'une quantité de gaz prédéfinie.

9. Procédé selon l'une des revendications précédentes, **caractérisé en ce que** la puissance de la pompe est augmentée après une période équivalant à la période dont le support (5) a besoin pour passer du capteur (16) au rotor (3) de la pompe (1).

10. Procédé selon l'une des revendications précédentes, **caractérisé en ce que** la puissance de la pompe est augmentée après écoulement d'une période prédéfinie suivant la première entrée de gaz détectée. (p.ex. une période supérieure à 3 s)

11. Procédé selon l'une des revendications précédentes, **caractérisé en ce que** la puissance de la pompe est pulsée pendant une période dépassant 3, de préférence dépassant 5 secondes.

12. Procédé selon l'une des revendications précédentes, **caractérisé en ce que** la pompe (1) est actionnée lors du priming de façon pulsatile.

13. Procédé selon l'une des revendications précédentes, **caractérisé en ce que** le gaz (25) est détecté par mesurage de la puissance absorbée de la pompe (1) par rapport à la puissance de sortie de celle-ci.

14. Procédé selon l'une des revendications précédentes, **caractérisé en ce que** le gaz (25) est détecté par mesurage de la température sur le palier (10, 11) du rotor (3).

15. Pompe à sang (1), notamment pompe centrifuge ou pompe diagonale, comportant un rotor (3), un boîtier (6) et un mécanisme d'entraînement (7), **caractérisée en ce qu'**elle comprend un détecteur de gaz (16, 21, 23) agissant sur le mécanisme d'entraînement (7) lors de la détection de gaz (25) pour augmenter temporairement la puissance de la pompe en fonction du gaz mesuré.

16. Pompe à sang selon la revendication 15, **caractérisée en ce que** le détecteur (16) est disposé dans une alimentation (2) vers la pompe (1).

17. Pompe à sang selon l'une des revendications précédentes de dispositif, **caractérisée en ce que** le détecteur (21) est disposé dans ou sur le boîtier de la pompe (1).

18. Pompe à sang selon l'une des revendications précédentes de dispositif, **caractérisée en ce que** le détecteur (23) est disposé sur le rotor (3) ou sur le palier (10, 11) du rotor.

19. Pompe à sang selon l'une des revendications précédentes de dispositif, **caractérisée en ce que** le détecteur (16, 21) est un capteur mesurant sans contact.

20. Pompe à sang selon l'une des revendications précédentes de dispositif, **caractérisée en ce que** le capteur du détecteur (16, 21, 23) n'émet pas de signal lors du transfert du support (5) et émet un signal lors de l'entrée d'air.
